# EUROPEAN PATENT SPECIFICATION

(11) **EP 4 128 019 B1**
(45) Date of publication and mention of the grant of the patent: **31.12.2025**
(21) Application number: 21714962.4
(22) Date of filing: 23.03.2021
(51) Int. Cl.: G06V 20/69, G06F 18/40, C12M 1/36, C12Q 1/04, C12M 1/26

(54) **MICROORGANIC DETECTION SYSTEM WITH WORKLISTS**
MIKROORGANISCHES DETEKTIONSSYSTEM MIT ARBEITSLISTEN
SYSTÈME DE DÉTECTION MICRO-ORGANIQUE AVEC DES LISTES DE TRAVAIL

(30) Priority: 26.03.2020 US 202063000319 P
(43) Date of publication of application: 08.02.2023
(73) Proprietor: Neogen Food Safety US HoldCo Corporation, Lansing MI 48912 (US)
(72) Inventor: DUGAN, Jessica B., Saint Paul, Minnesota 55133-3427 (US); VANDERHILL, Shannon E., Saint Paul, Minnesota 55133-3427 (US); WANG, Geng, Saint Paul, Minnesota 55133-3427 (US)
(74) Representative: Mathys & Squire
(86) International application number: PCT/IB2021/052398
(87) International publication number: WO 2021/191793

(56) References cited:
- WO-A2-2008/143849
- GB-A- 2 407 193
- US-A1- 2011 151 501

## Description

### BACKGROUND

The food industry routinely monitors the levels of indicator groups of microorganisms. These microorganisms are considered quality and hygienic indicators and can pose health hazards to humans. A common method of quantifying indicator microorganisms is counting colony forming units. The counting can be performed by first culturing target microorganisms on culture devices (e.g., dry film or agar plates), then counting the colony forming units. Colony counts may be done at multiple stages including on incoming materials, in process product, finished product, and environmental samples taken from within the food processing environment.

The colony count data can be used for many purposes such as to meet regulatory requirements, shelf life claims, reduce spoilage, ensure hygiene of processes and facilities, ensure effectiveness of process steps (cooking, pasteurization, decontamination, etc.), or ensure final product is free from contamination which could cause illness.

Counting colonies on growth media and interpretation of these counts can be very complex and can greatly benefit from improvements in colony enumeration devices and software. The unique challenges and requirements in this space which make this a complex effort include A) Media interpretation challenges and accuracy requirements, B) Physical factors of the media, and C) Complexity of data.

Laboratories, specifically labs that utilize high volumes of growth media to monitor the levels of indicator groups of microorganisms, currently have various manual methods for organizing and communicating which samples/tests need to be processed in a given timeframe (such as but not limited to a work shift, a day, a week or a month, etc.). Examples of manual methods include the use of paper lists, printed spreadsheets, bulletin boards, emails or output from a data management system.

An example of a prior art document teaching a modular system for detecting micro-organisms is US 2011/151501 A1, published on 23-06-2011.

### BRIEF SUMMARY

The present disclosure relates to a method as defined in claim 1 and a system as defined in claim 10.

Additional aspects of the present disclosure also can relate to a computing apparatus. The computing apparatus can include a display device configured to display a user interface and an input device communicatively coupled to the user interface to allow a user to interact with the user interface. The computing apparatus can include a processor, and a memory storing instructions that, when executed by the processor, configure the computing apparatus to receive, from the user interface, a dilution profile that includes a plurality of profile fields relating to a series of test instances for a plurality of culture devices. The processor can further store the dilution profile in the memory.

The memory can store instructions that configure the computing apparatus to receive a request to form a worklist for reading the plurality of culture devices via the user interface. The worklist has a first test instance with a different plate type and a dilution factor from a second test instance. The worklist can include a plurality of worklist fields that correspond to at least some of the plurality of profile fields in the dilution profile.

The memory can store instructions that configure the computing apparatus to determine that the dilution profile applies to the worklist and populate, responsive to the determination that the dilution profile applies, at least some of the plurality of worklist fields with the plurality of profile fields in the dilution profile. The computing apparatus can be configured to perform at least one action responsive to the populating.

An additional aspect of the present disclosure can relate to a plate reader having image processing circuitry. The computing apparatus can performing the at least one action by transmitting instructions to the plate reader, using the worklist, that causes the image processing circuitry of the plate reader to capture an image of a culture device that is identified in the worklist.

The above summary of the present disclosure is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

Additional details of these and other embodiments are set forth in the accompanying drawings and the description below. Other features, objects and advantages will become apparent from the description and drawings, and from the claims.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 illustrates a method 100 in accordance with one embodiment.
FIG. 2 illustrates a user interface 200 in accordance with one embodiment.
FIG. 3 illustrates a user interface 300 in accordance with one embodiment.
FIG. 4 illustrates a method 400 in accordance with one embodiment.
FIG. 5 illustrates a user interface 500 in accordance with one embodiment.
FIG. 6 illustrates a user interface 600 in accordance with one embodiment.
FIG. 7 illustrates a method 700 in accordance with one embodiment.
FIG. 8 illustrates a data scheme 800 in accordance with one embodiment.
FIG. 9 illustrates a user interface element 900 in accordance with one embodiment.
FIG. 10 illustrates a method 1000 in accordance with one embodiment.
FIG. 11 illustrates a method 1000 in accordance with one embodiment.
FIG. 12 illustrates a data structure 1200 in accordance with one embodiment.
FIG. 13 illustrates a simplified microorganic detection system 1300 in which a server 1304 and a client device 1306 are communicatively coupled via a network 1302.
FIG. 14 is an example block diagram of a computing apparatus 1400 that may incorporate embodiments of the present invention.

### DETAILED DESCRIPTION

Manual methods of colony enumeration can be cumbersome to track and monitor, easy to misplace and/or easy to ignore (contributing to inefficiency, mistakes and breakdowns in recordkeeping and traceability).

Aspects of the present disclosure can allow a user, via a user interface, to build, schedule, track and maintain records for worklists in the same microorganic detection system. Thus, a plate reader used to process a plurality of culture devices can be communicatively coupled to a worklist on a user interface on a computing apparatus. Aspects of the present disclosure can improve user workflow, increases lab and worker efficiency and contributes to better traceability of records for the given lab.

An additional aspect of the present disclosure can allow the user to use a dilution profile to populate at least some data fields of the worklist. The dilution profile can also include the product specifications for a microorganic sample type in the plate reader. By linking the dilution profile, the worklist, and the plate reader, the overall performance of the microorganic detection system can be improved by minimizing user inputs (which incur less waiting for the user).

An additional aspect of the present disclosure is the ability to leverage a culture device identification (e.g., barcodes with encoded meta data) to create worklists without manual data entry and the ability to more easily track if a given culture device has been fed into the device.

Additional aspects of the present disclosure can also allow a user to schedule reminders which can alert users when a certain batch of culture devices needs to be completed. In at least one embodiment, a worklist can be visible across user profiles. Thus, if a laboratory uses multiple profiles on a computer and/or computing apparatus, then the reminders, worklist, and results library is available to each user.

As described further herein, a worklist is a way for laboratories to streamline daily microbiological testing workflow with the assistance of a plate reader and associated laboratory information management system (LIMS) running on a computing apparatus, specifically in planning, running, and managing microbiological tests. The computing apparatus can use multiple data structures that help a user to construct a worklist. These data structures can serve as templates that can be used to define a worklist. This saves time, reduces the potential for errors and creates efficiency within the lab environment.

Further, the computing apparatus can automatically generate specifications for each run of the test according to the template component of user's choice. Once a worklist is constructed, a lab technician and the instruments controlled by the computing apparatus can use the worklist as instructions to choreograph the running of a sequence of culture devices.

In at least one embodiment, the worklist can be set up by a computing apparatus which can be used by the plate reader effectively use the data fields of a test instance to analyze sample data relating to the test instance.

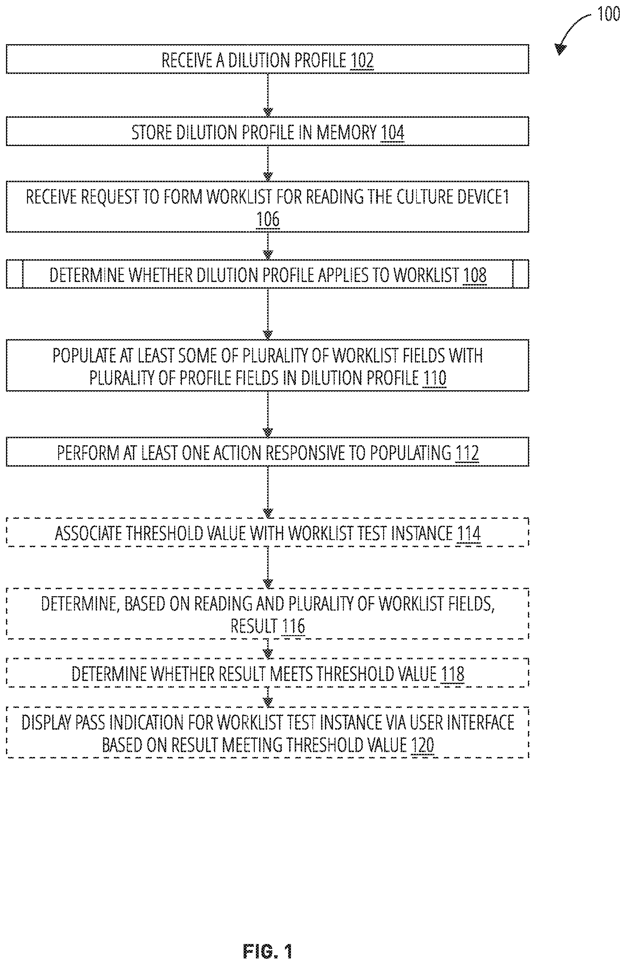

FIG. 1 illustrates a computer-implemented method 100 of using a dilution profile to form a worklist. The worklist can be used by a computing apparatus to analyze culture devices from a plate reader . In at least one embodiment, image processing circuitry (e.g., the camera, barcode reader, etc.) and illumination circuitry of the plate reader can be affected by the worklist. For example, the worklist can determine when data from the plate reader is transmitted or the worklist can determine when the camera is activated. A user can interact with the worklist via a user interface of the computing apparatus.

In block 102, the computing apparatus receives, from a user interface on a display device of a computing apparatus, a dilution profile. The dilution profile can include a plurality of profile fields. Each profile field is a data field related to a property of a culture device that is processed by a plate reader. A dilution profile can represent a series of test instances or a single test instance for the culture device. For example, the dilution profile can be structured data (e.g., stored in a relational data store) and can have a plurality of columns that represent, for example, culture device identification, plate type, dilution, product specification, and combinations thereof. A profile field for a test instance can be associated with any of the plurality of columns. A test instance can correspond to a row in structured data.

In at least one embodiment, the dilution profile is created by the user via the user interface. For example, the user can fill in the plurality of profile fields for a series of test instances to create the dilution profile. In at least one embodiment, the series of test instances can be performed by the plate reader in a specific order. In other embodiments, the series of test instances can be indeterminate.

In at least one embodiment, the dilution profile can be received by the computing apparatus from a data store. For example, the dilution profile can be present in the data store and fetched by the computing apparatus via a network.

In block 104, the computing apparatus optionally stores the dilution profile in the memory. The memory can refer to various hardware implementations used to store data either temporarily such as RAM, cache, or permanently such as a hard disk, solid state drive, tape drive, or combinations thereof.

In block 106, the computing apparatus receives a request to form a worklist for reading the culture device. The reading can be performed the plate reader. For example, image processing circuitry and illumination circuitry of the plate reader can capture an image of a culture device. The culture device can also include a nutrient medium and a microorganic sample. For example, a microorganic sample from a food product can be applied to multiple culture devices which each form a test instance to be read by the plate reader. The series of test instances are read according to the worklist.

In at least one embodiment, the worklist can have a first test instance with a different plate type and a dilution factor from a second test instance. For example, the first test instance can have an all count plate type with a 1:100 dilution factor and the second test instance can have a coliform count plate type with a 1:10 dilution factor. In at least one embodiment, the worklist can have a plurality of worklist fields that correspond to at least some of the plurality of profile fields in the dilution profile. For example, the worklist can have more worklist fields than the dilution profile has profile fields, or vice versa.

In subroutine block 108, the computing apparatus can determine whether (or that) the dilution profile applies to the worklist. The computing apparatus can determine whether any portion of the worklist can be autofilled (e.g., applied in an automated manner such as a form complete or drop-down options in response to a filled data field) with the dilution profile as described further herein. In at least one embodiment, the computing apparatus can receive an indication from a user, via the user interface, that the dilution profile applies to the worklist when the worklist is being created. For example, during the creation of a worklist in block 106, the user can be presented with an option to apply the dilution profile. If selected, then subroutine block 108 can be satisfied.

In block 110, the computing apparatus can populate, responsive to the determination that the dilution profile applies, at least some of the plurality of worklist fields with the plurality of profile fields in the dilution profile. For example, if there are more worklist test instances (having a plurality of worklist fields) than profile test instances (having a plurality of profile fields), then the computing apparatus can populate the worklist fields with profile fields corresponding to the worklist test instances and request more information regarding the worklist test instances that do not correspond to the profile test instances.

Conversely, if there are more profile test instances than worklist test instances, then the method 100 can stop and the computing apparatus can provide a prompt that the dilution profile does not apply, or the computing apparatus can present the dilution profile (e.g., all of the profile test instances) and allow the user to select a series of test instances that apply to the worklist. The computing apparatus can use the series of test instances selected by the user to populate at least some of the worklist data fields.

In block 112, the computing apparatus performs an action responsive to the populating. In at least one embodiment, the computing apparatus can cause the plate reader to load a culture device (e.g., by directing the user or autoloader attachment), read the culture device (e.g., with image processing circuitry of the plate reader), and transmit sample data (including the image) from the plate reader to the computing apparatus.

In at least one embodiment, the computing apparatus can further receive a time condition from a user, via the user interface, to begin reading the culture device. Once a time condition is satisfied, then the plate reader can cause or allow the culture device to be read. For example, the time condition can refer to a particular date and time that a culture device will be run. Once the time condition approaches, then the plate reader can automatically load, via an autoloader attachment, the culture device to be read by the plate reader or otherwise receive the sample data from the plate reader. In at least one embodiment, the plate reader can allow the culture device to be read at any time and only transmit the sample data after the time condition is satisfied. In at least one embodiment, the computing apparatus can generate an alert within the user interface at the time condition to allow the user to initiate loading of the culture device.

The action can also include the computing apparatus notifying the user via the user interface that the culture device corresponding to the worklist test instance is being or is scheduled to be read. For example, the computing apparatus can notify the user on the progress of the worklist.

In at least one embodiment, the action can include the computing apparatus or plate reader verifying an identity of the culture device to ensure that the culture device corresponds to a specific worklist test instance. For example, the computing apparatus includes determining or verifying whether a plate type of the culture device corresponds or otherwise matches a plate type from the worklist test instance by determining the plate type from the culture device. This can occur via optical recognition of elements from the culture device (such as reading a label), or can occur by scanning an identification element with built-in identification such as a barcode, or RFID.

After the computing apparatus determines that the plate type of the culture device (received from the plate reader) corresponds to the worklist test instance, the plate reader can read the culture device. In at least one embodiment, reading the culture device can include reading a plurality of culture devices each associated with a test instance. For example, if there is no match, then the plate reader can lock out or ignore sample data from the culture device.

In at least one embodiment, the at least one action can be reading the culture device to produce the sample data. The sample data can correspond to at least one worklist test instance.

In at least one embodiment, block 114 through block 120 can be optional and involve determining if a result (e.g., from the plate reader reading the culture device in block 112) meets a product specification.

For example, in block 114, the computing apparatus can associate a threshold value with a worklist test instance. The threshold value can be a product specification for a food product. The product specification can be associated with the worklist test instance.

In block 116, the computing apparatus can determine, based on the reading and the plurality of worklist fields, a result. The result can be based on the sample data which can be obtained from the plate reader reading the culture device in block 112. The sample data can be analyzed according to the plurality of worklist fields for a test instance. For example, a dilution factor and a plate type can modify the raw colony counts from the sample data to further determine the type of microorganism and the colony count of the microorganism (which can be a result therein).

In block 118, the computing apparatus can determine whether the result meets the threshold value. As discussed herein, the threshold value can be related to the product specification for a given food product sample. If the result meets the threshold value, then, in block 120, the computing apparatus can display a pass indication for the worklist test instance via the user interface. For example, the product specification for pasteurized milk depends on the grade. The standard for USDA grade A milk is less than 750,000 somatic cells per milliliter. In this example, the threshold value is based off of the somatic cell concentration. If the somatic cell count is 400,000 cells per milliliter, then the result meets the threshold value for grade A milk. If the somatic cell count is 800,000 cells, then the result would not meet the threshold value but may still qualify as a lower grade of milk. In at least one embodiment, the computing apparatus can determine which product specification is satisfied and present that to the user. For example, the computing apparatus can determine that the milk sample, while not being grade A, qualifies as a lower grade of milk which can reduce food waste.

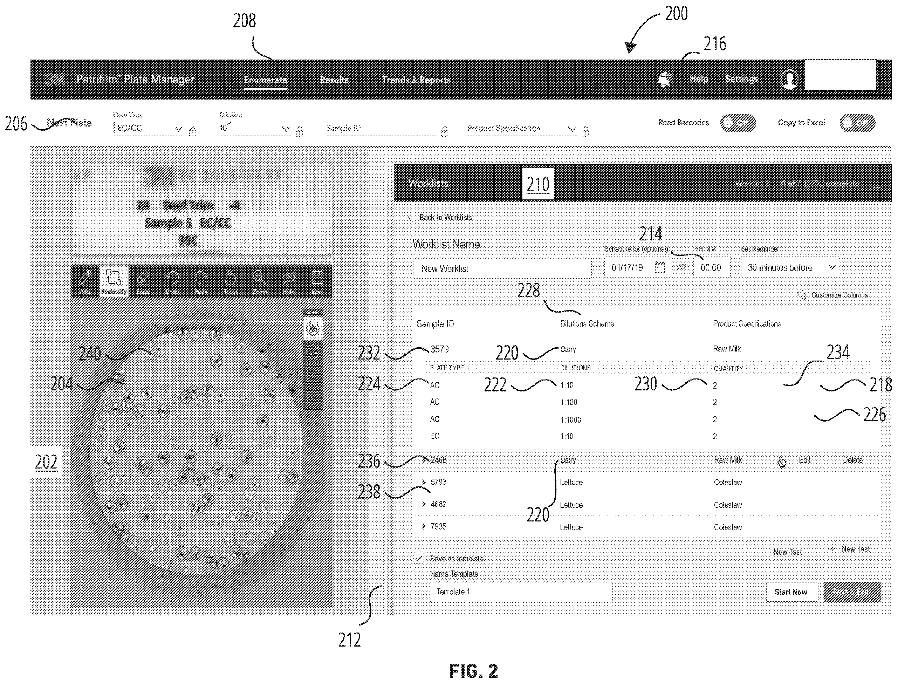

FIG. 2 illustrates an example view of a user interface 200. The user interface 200 can include ribbon tabs 208, ribbon content area 206, image section 202 (showing image 204 which can be part of the sample data) and main content area 212. The image 204 can be of a culture device and include a colony forming unit 240 shown highlighted thereon. Within main content area 212, a worklist can be created in worklist section 210.

The worklist section 210 illustrates the creation of a single worklist. The worklist section 210 can include the name of the worklist and a time condition 214 of the worklist. The time condition 214 can be associated with notification indication 216.

A worklist can include a plurality of samples (e.g., sample 232 and sample 236). For example, a sample can refer to a microorganic sample from a single source (e.g., a batch of a food product) or can refer to a food matrix (which are samples collected according to a protocol (not necessarily from the same source, but can be of the same type)). The microorganic sample can be applied to a plurality of culture devices and read as a series of test instances 218. Thus, sample 232 can be associated with a series of test instances 218. As shown, the individual test instances for a given sample 232 are nested and grouped by dilution underneath a main header for ease in viewing and planning all test instances associated with a given microorganic sample.

In worklist section 210, a plurality of worklist fields 230 are shown for sample 232. As shown, the plurality of columns 228 are different for the main header versus the subheaders. For example, the main headers are shown as sample identification (ID) representing sample 232, dilution profile 220, and product specification while the subheaders are shown as plate type 224, dilution factor 222, and quantity. A single test instance can have a test instance identifier 234. In at least one embodiment, the test instance identifier 234 can be numbered or simply indicated with a visual separation between test instances as shown. A plurality of profile fields 226 can match with at least some of the plurality of worklist fields 230.

In worklist section 210, the sample 236 is shown having a careted, unexpanded section using the same dilution profile 220 as sample 232. For example, sample 232 and sample 236 can both use the same dilution profile 220. The worklist section 210 can also have samples that use different dilution profiles. For example, sample 238 can use a dilution profile for lettuce different from dilution profile 220. Thus, a worklist can include a plurality of dilution profiles with different dilution profiles existing in the same worklist.

In at least one embodiment, the worklist can set the plate types and dilution factors for the plate reader to read a plurality of culture devices. After each culture device is read by the plate reader according to the worklist, the results for the culture device (including the image 204) can be displayed in the image section 202. Further, the worklist can be linked to the autoloader attachment of the plate reader to perform the analysis in an automated manner.

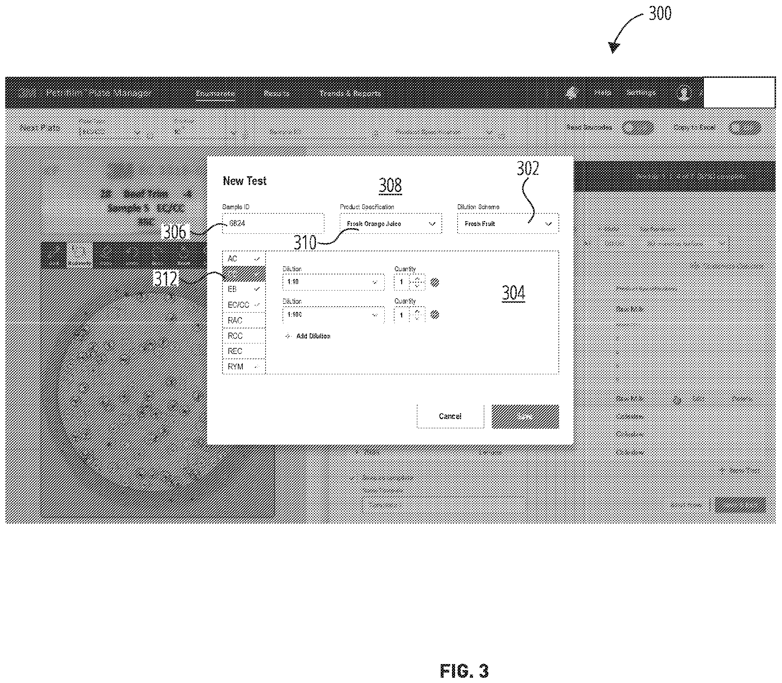

FIG. 3 illustrates user interface 300. The user interface 300 can be like user interface 200 in FIG. 2 except user interface 300 can include a request menu 308. In at least one embodiment, the request menu 308 can be a pop-up menu that is triggered upon editing any portion of the existing worklist or upon creation of a new worklist (e.g., block 106 in FIG. 1).

User interface 300 can illustrate an example process for adding plurality of profile fields 304 (e.g., including a new sample ID) to a worklist. In user interface 300, the user can choose to add tests manually, dilution profiles, or combinations thereof.

For example, a request menu 308 can include the sample data field 306 which can be manually entered via a text field. A user can select the product specification data field 310 and the dilution profile data field 302 (e.g., by a drop-down menu). Upon selection, the computing apparatus can load a plurality of profile fields 304 onto the worklist. Although dilution profile data field 302 and product specification data field 310 are shown to be selected independently, the dilution profile data field 302 and product specification data field 310 can be linked. For example, selecting fresh fruit in dilution profile data field 302 can limit the options presented to the user in product specification data field 310 (which shown as being limited to fresh orange juice).

The plate type data field 312 can be populated based on the dilution profile data field 302 selected. For the given coliform count plate type, two dilutions can be performed on two culture devices. In at least one embodiment, the computing apparatus can cause the autoloader attachment to automatically perform the dilutions prior to being read by the plate reader.

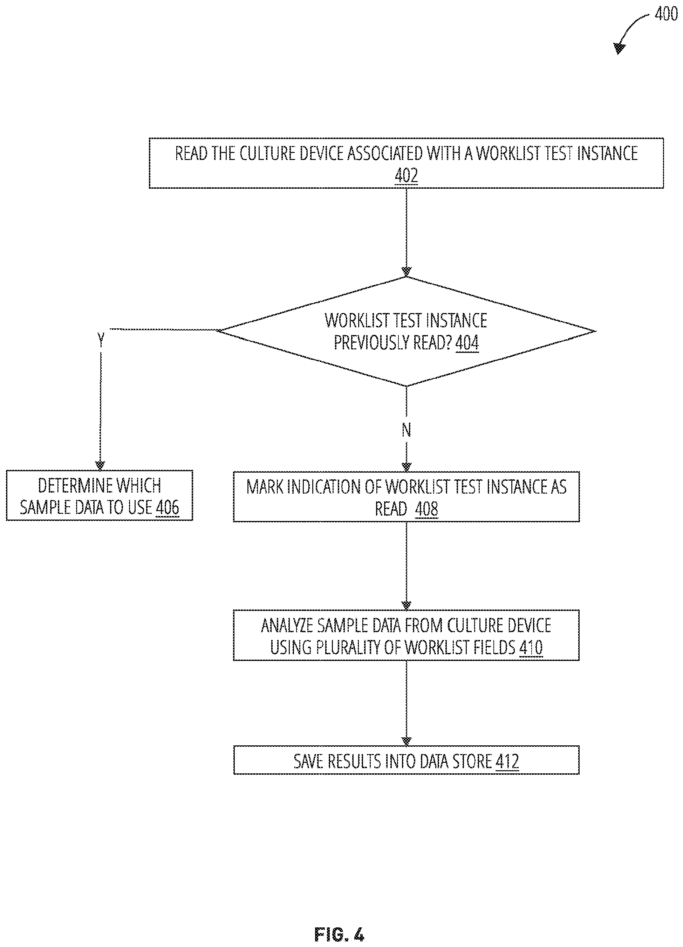

FIG. 4 illustrates a method 400 for tracking the worklist test instances in the worklist. Method 400 can be an embodiment of block 112. In block 402, the computing apparatus can cause the plate reader to read/capture the culture device associated with the worklist test instance. For example, the computing apparatus can trigger the plate reader to load the culture device, capture an image of the culture device, and/or transmit the image/sample data to the computing apparatus. The sample data can further include any identification of the culture device (e.g., using the built-in identification of the culture device).

In decision block 404, the computing apparatus can determine whether the worklist test instance was previously read. For example, if the identification of the culture device indicates that the culture device read has been read previously, then the computing apparatus determines which sample data to use (i.e., the first sample data or the second sample data) in block 406.

In at least one embodiment, block 406 can include verifying that the culture device is acceptable to produce a reading. For example, the computing apparatus can analyze the incubation times of the culture device to determine if the culture devices meet a product specification for incubation time. Alternatively, the computing apparatus can prompt a user to select which sample data to use.

If the worklist test instance was not previously read, then the computing apparatus can mark an indication of the worklist test instance as read in response to the reading in block 408.

In block 410, the computing apparatus can analyze the sample data from the culture device using the plurality of worklist fields to produce a result described herein.

In block 412, the computing apparatus can optionally save the result into memory or a data store to be accessed later (e.g., used in the determination in decision block 404).

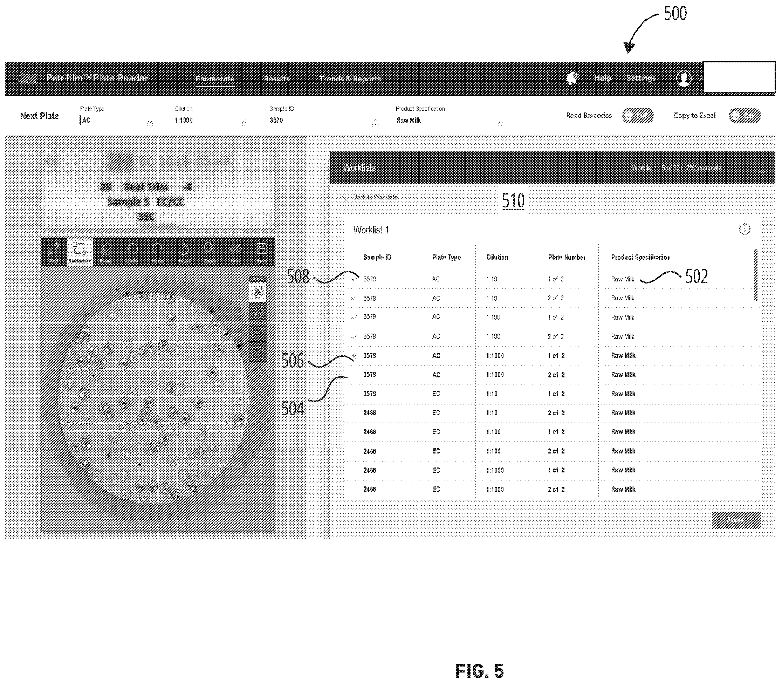

FIG. 5 illustrates an example user interface 500 that illustrates concepts from method 400. For example, user interface 500 shows an example view of a worklist progress section 510. The worklist progress section 510 can illustrate the progress of the series of test instances 502 relative to a completion of the worklist as a whole. As shown, the series of test instances 502 all use the same product specification. In at least one embodiment, the series of test instances 502 can also use the same dilution profile. The worklist progress section 510 can display a progress marker 508 (e.g., check and dot icons) for each completed worklist test instance. Once the test instance of a culture device is marked as complete by progress marker 508, then the test instance may not read in a subsequent instance (i.e., if the same culture device is placed in the plate reader). In at least one embodiment, the worklist progress section 510 can display progress marker 506 for worklist test instances that are in progress. In at least one embodiment, an unread test instance 504 can be unmarked.

The user can pause any of the series of test instances within the worklist. Each test instance is shown as occupying a row in the worklist progress section 510 versus being nested underneath a main heading as in user interface 200 herein. This arrangement can further aid in feeding and tracking, and also reduce potential errors.

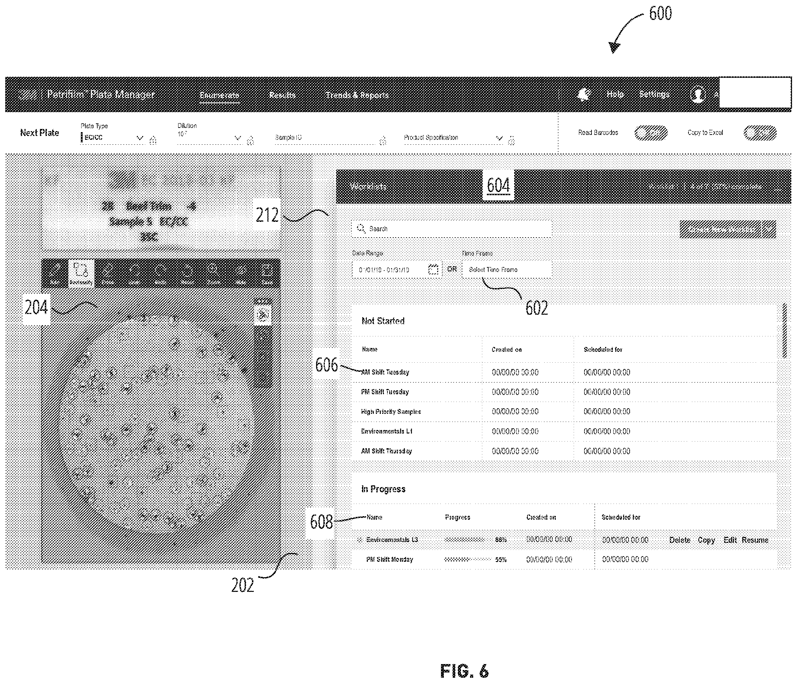

FIG. 6 illustrates an example user interface 600. The user interface 600 can have a worklist schedule window 604. In at least one embodiment, the worklist schedule window 604 can transition between an open window and closed window based on a selection within the main content area 212 and allow the user to toggle the worklist to create and manage the worklist process contemporaneously with reviewing the image 204 on the image section 202.

The worklist schedule window 604 can allow a user to schedule a plurality of worklists. For example, the user can specify a time condition 602 where when the culture devices corresponding to the worklist will be read by the plate reader. The worklist schedule window 604 shows plurality of worklists 606 which are not yet started and plurality of worklists 608 which are in progress.

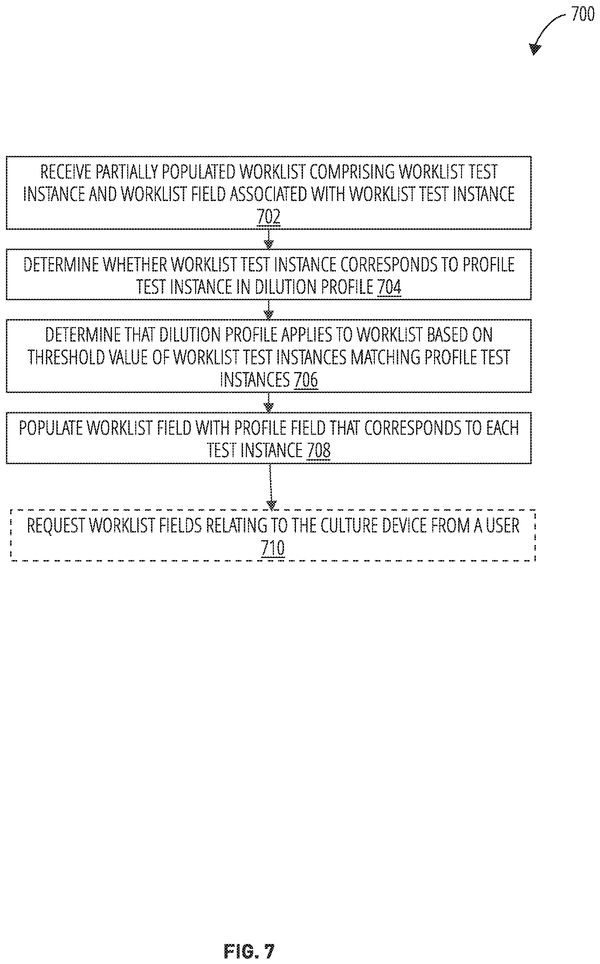

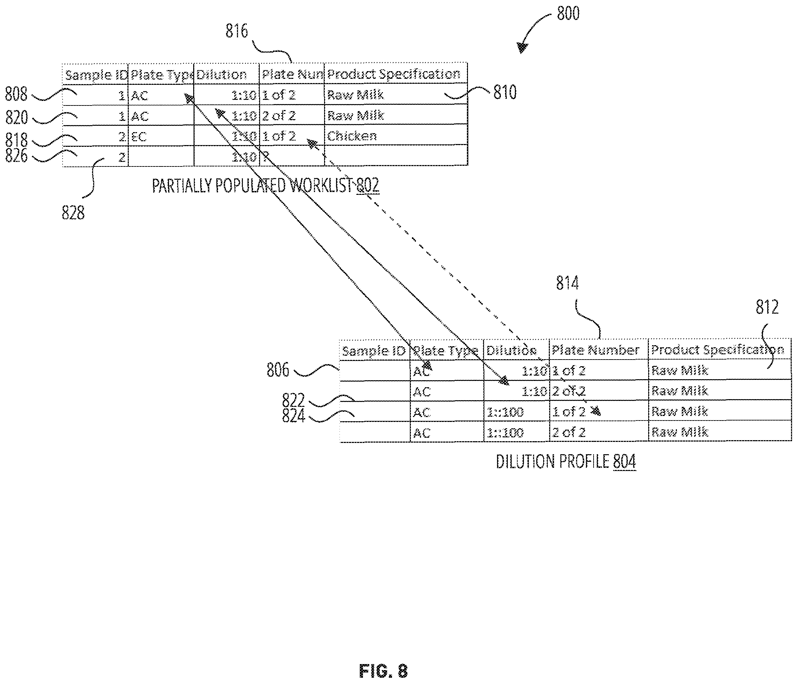

FIG. 7 illustrates an embodiment of a method 700 of determining whether a dilution profile applies to the worklist. In at least one embodiment, method 700 can be an embodiment of subroutine block 108 in FIG. 1. Aspects of method 700 will be explained with reference to data scheme 800 in FIG. 8. In at least one embodiment, the method 700 can also include autofilling a worklist based on input into the worklist.

In block 702, the computing apparatus can receive a partially populated worklist 802. The partially populated worklist 802 can include a worklist test instance 808 and a worklist field associated with the worklist test instance. In at least one embodiment, the partially populated worklist 802 can include a plurality of worklist test instances and a plurality of worklist fields. The number of worklist test instances can correspond to the partially populated worklist 802 and can be generated by a textual input from an input device. For example, a user can create part of a worklist by typing in the criteria for the first few fields of a test instance.

The partially populated worklist 802 or the dilution profile 804 can include a plurality of profile fields 812 or a plurality of worklist fields 810 that can be associated with a plurality of columns of a data type. For example, the plurality of worklist fields 810 can be categorized by a plurality of columns 816. The plurality of profile fields 812 can be categorized by plurality of columns 814. In another example, at least one of the plurality of worklist fields 810 is metadata.

As shown, the plurality of columns 814 includes an optional test instance identifier, and the plate type and dilutions for each test instance identifier. A column from the plurality of columns 816 can include a worklist sequence 828 that represents a reading order of a culture device.

In block 704, the computing apparatus determines whether the worklist test instance corresponds to a profile test instance in the dilution profile. The worklist test instance can correspond to a profile test instance when at least one of the plurality of profile fields for a profile test instance matches at least one of the plurality of worklist fields for a worklist test instance. In at least one embodiment, the worklist test instance can correspond to a profile test instance if a majority of the plurality of profile fields for a profile test instance matches a majority of the plurality of worklist fields for one or more worklist test instances. In at least one embodiment, the worklist test instance can determine if all of the (filled) plurality of worklist fields matches all of the (filled) plurality of profile fields for a single test instance.

For example, the profile test instance 806 can correspond to worklist test instance 808 because all of the plurality of worklist fields 810 matches plurality of profile fields 812 for each instance. Worklist test instance 820 can correspond to profile test instance 822. However, worklist test instance 818 does not correspond to profile test instance 824 because only the sample ID data field matches between the worklist field and profile field. As shown, none of the other profile fields match the worklist fields.

In at least one embodiment, selected columns can have a higher weight relative to other columns. For example, the plate type column can have a higher weight than the plate number column. Thus, since the data field in the plate type column does not match between the worklist test instance 818 and profile test instance 824, then the two test instances do not correspond to one another.

In block 706, the computing apparatus can determine that the dilution profile applies to the worklist based on a threshold value of worklist test instances matching the profile test instances. For example, determining whether the dilution profile applies to the worklist can be based on the number of profile test instances that match the worklist test instances, or vice versa. For example, in data scheme 800, if the threshold value is three worklist test instances, then because only two worklist test instances match with two profile test instances, then the computing apparatus can determine that the dilution profile 804 does not apply to the partially populated worklist 802. However, if the threshold value is two worklist test instances, then the dilution profile 804 would apply to the partially populated worklist 802.

In block 708, the computing apparatus can populate a worklist field with a profile field that corresponds to each test instance. For example, the profile fields for profile test instance 806 and profile test instance 822 can be used to populate the worklist fields for worklist test instance 808, and worklist test instance 820 respectively. In at least one embodiment, the populating can be in response to the computing apparatus determining that the dilution profile applying to the worklist in block 706. For example, if there are no matches, then there is no populating. In at least one embodiment, the threshold value can be one test instance matching in block 706, which can result in each profile test instance populating on a worklist test instance basis. For example, after a worklist field of worklist test instance 808 is entered, then the computing apparatus can populate the remaining worklist fields for worklist test instance 808. In another example, after worklist test instance 808 is entered and profile test instance 806 corresponds to worklist test instance 808, then worklist test instance 820 can be autofilled based off of profile fields for profile test instance 822.

In block 710, the computing apparatus can optionally request any worklist fields relating to the culture device from a user, via the user interface, for any missing worklist fields from the partially populated worklist. For example, if the non-corresponding profile test instances (e.g., profile test instance 824) do not match the partially populated worklist 802 (e.g., worklist test instance 818), then the computing apparatus can prompt the user to fill in worklist test instance 818 and worklist test instance 826. Even if all of the profile test instances correspond to some of the worklist test instances, the remaining worklist test instances can be filled by the user or filled in based on another dilution profile.

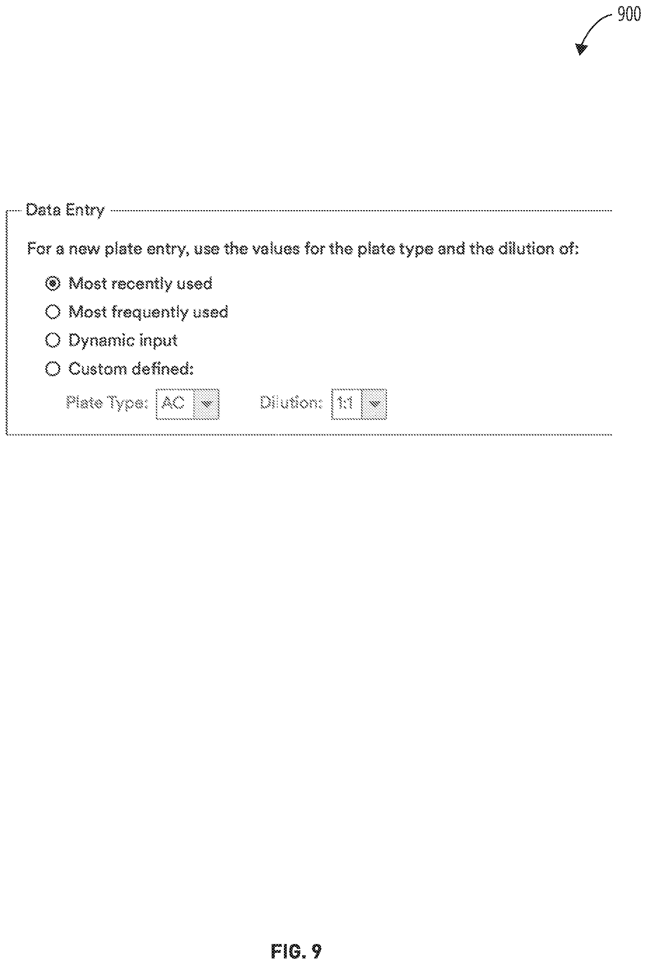

FIG. 9 illustrates an embodiment of a user interface element 900 that is a variation of block 110. The user interface element 900 can be a part of the user interface described herein. As shown, the user can select automated data entry which will cause the computing apparatus to predict the metadata information for a new culture device, given the input history of a specific user, or all the users in a lab. The computing apparatus can learn to adapt to user input behavior over time (for example, by accounting for metadata that is the most frequently used, most recently used, or "dynamic input" (for example, once the user selects a plate type, the method will predict and selects dilution for the user), or custom defined.

The automated data entry can be based off of the method 700 described above or another machine learning mode. As the user types a worklist field and the worklist field corresponds to at least one profile field, then the computing apparatus can provide a next profile field as the next worklist field. The options of next profile fields can be presented as a dropdown menu for the user to select from.

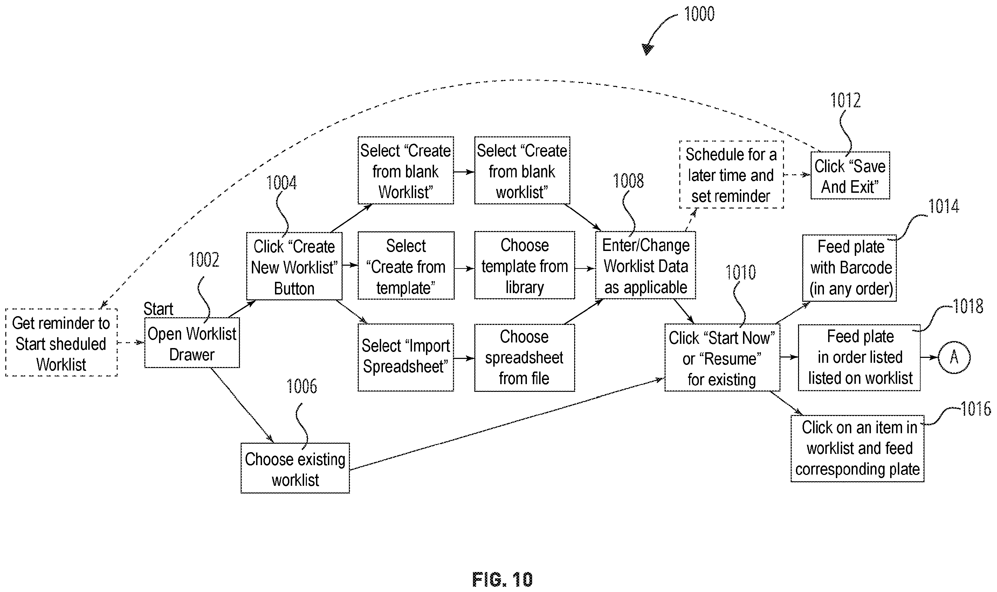

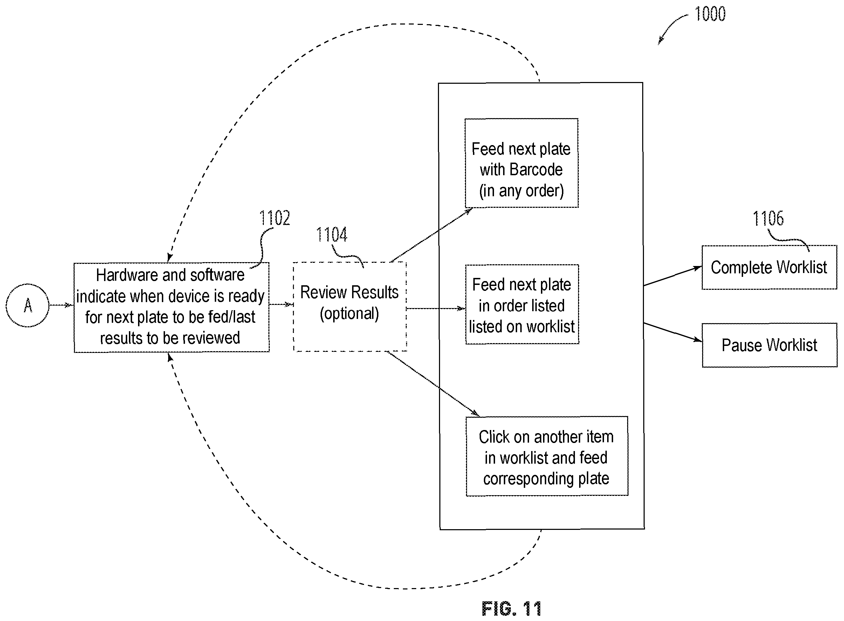

FIG. 10 and FIG. 11 illustrate an example method 1000 of interacting with the user interface. For example, via the user interface and the input devices, a user can open a worklist drawer in block 1002. The user can create a new worklist in block 1004 or choose an existing worklist in block 1006.

If a new worklist is created, then a user can create a new worklist for receiving sample data from a plate reader or use an existing worklist. The new worklist can rely on batch-feeding culture devices with pre-populated metadata into the computing apparatus. The computing apparatus can use multiple data structures that help a user to construct a worklist. These data structures can serve as templates that can be used to define the worklist. Once a worklist is constructed, a user and the instruments controlled by the system use the worklist as instructions to choreograph the running of a series of tests.

The user can specify all the required information for each run. A metadata list can also be imported from external data source such as customer's LIMS. In block 1008 the user can choose to "Save & Exit" in block 1012 or click the "Start Now" button in block 1010. If the user chose to save and exit, then user also has the option to schedule the worklist for a later time and set a reminder. The user may receive a notification from the computing apparatus at a designated time.

If the user selects "Start Now" in block 1010, then the computing apparatus can support three distinct workflows for feeding growth medias. For example the user or plate reader can feed growth media with a barcode in any order in block 1014, feed culture device or plate in the order of the worklist in block 1018, or click on an item in the worklist and feed the corresponding culture device in block 1016.

After feeding the culture device according to one of the workflows, the plate reader can process the culture device and displays information for each culture device without the user entering metadata again. In block 1102, when culture device enumeration is complete, the instrument prompts the user with the information for the next culture device. The user can choose to close the worklist panel and review the growth media results in block 1104. Once the worklist completes in block 1106, then the computing apparatus can communicate a completion status to the user. In at least one embodiment, the computing apparatus can additionally inform the user of the status of each worklist in progress and allow the pausing and restarting of a worklist.

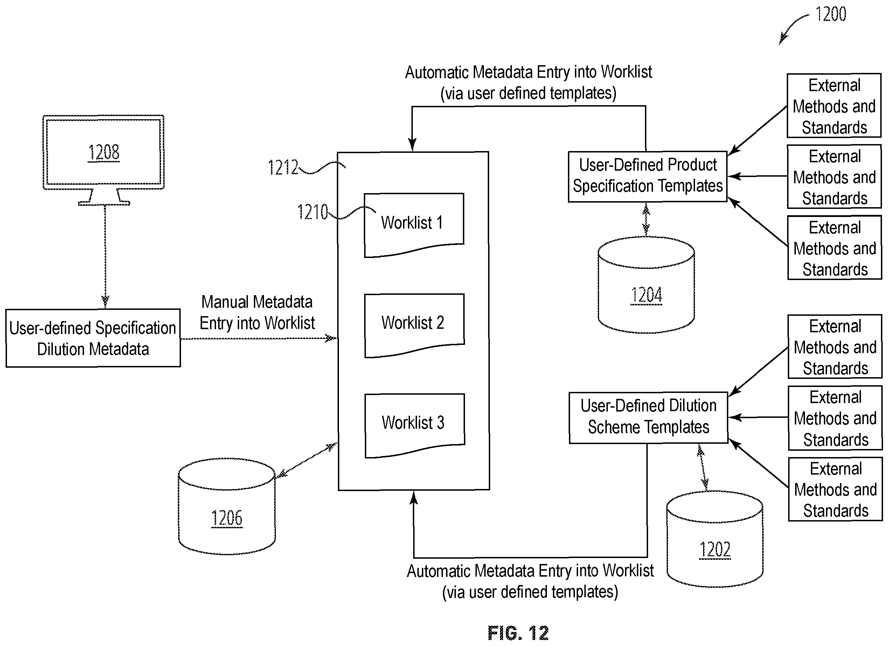

FIG. 12 illustrates a data structure 1200 for use with a worklist. The user interface 1208 can be used to create user-defined product specifications and dilution profiles (e.g., metadata relating to profile fields) that form part of a worklist 1210. A plurality of worklists 1212 can be stored in data store 1206 which can be either internal to the computing apparatus or accessed via a network by the computing apparatus.

In at least one embodiment, the computing apparatus can access data store 1204 which can store the user-defined product specifications based on external methods and standards. For example, one of the product specifications can be linked to a United States Department of Agriculture standard, that when updated, will automatically update the data store 1204 and the associated worklist. Similarly, the computing apparatus can access data store 1202 which can store user-defined dilution scheme templates. The data store 1202 and data store 1204 can be linked with the plurality of worklists 1212.

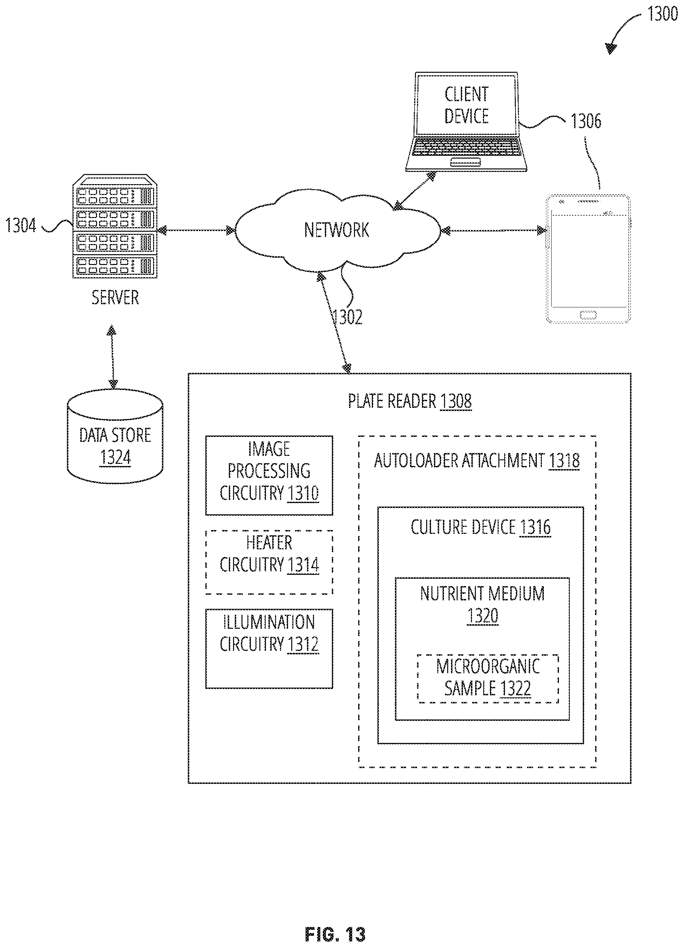

FIG. 13 illustrates a microorganic detection system 1300 in which a server 1304 and a client device 1306 are connected to a network 1302.

In various embodiments, the network 1302 may include the Internet, a local area network ("LAN"), a wide area network ("WAN"), and/or other data network. In addition to traditional data-networking protocols, in some embodiments, data may be communicated according to protocols and/or standards including near field communication ("NFC"), Bluetooth, power-line communication ("PLC"), and the like. In some embodiments, the network 1302 may also include a voice network that conveys not only voice communications, but also non-voice data such as Short Message Service ("SMS") messages, as well as data communicated via various cellular data communication protocols, and the like.

In various embodiments, the client device 1306 may include desktop PCs, mobile phones, laptops, tablets, wearable computers, or other computing devices that are capable of connecting to the network 1302 and communicating with the server 1304, such as described herein.

In various embodiments, additional infrastructure (e.g., short message service centers, cell sites, routers, gateways, firewalls, and the like), as well as additional devices may be present. Further, in some embodiments, the functions described as being provided by some or all of the server 1304 and the client device 1306 may be implemented via various combinations of physical and/or logical devices. However, it is not necessary to show such infrastructure and implementation details in FIG. 13 in order to describe an illustrative embodiment. In at least one embodiment, the server 1304 can be communicatively coupled to a data store 1324 such that data from the client device 1306 can be transmitted to the data store 1324 through the server 1304.

In at least one embodiment, the microorganic detection system 1300 can include a plate reader 1308 that is communicatively coupled to the client device 1306. The plate reader 1308 can be configured to read a culture device 1316. Examples of plate readers (also referred to as colony counters) are commercially available from 3M (Saint Paul, MN), Synbiosis (UK), or Interscience (France). In at least one embodiment, the plate reader 1308 can be directly connected to the client device 1306 without the use of a network 1302. Aspects of the user interface can be integrated onto the plate reader 1308 itself.

The plate reader 1308 can have multiple subsystems to aid in the reading of culture device 1316. For example, the plate reader 1308 can include image processing circuitry 1310 to capture images of the culture device 1316, an optional heater circuitry 1314 to incubate the culture device 1316, and an optional autoloader attachment 1318 to automatically feed multiple culture devices 1314 without user intervention. The image processing circuitry 1310 can be configured to provide the images to the client device 1306 for analysis. The image processing circuitry 1310 can work in conjunction with illumination circuitry 1312. The illumination circuitry 1312 can include various lights and controllers to illuminate the culture device to be captured by the image processing circuitry 1310.

The culture device 1316 can have growth compartment having a nutrient medium 1320 contained therein. A microorganic sample 1322 can be obtained from a sample of interest such as a food sample. The microorganic sample 1322 can be applied to nutrient medium 1320 such that the microorganism can proliferate.

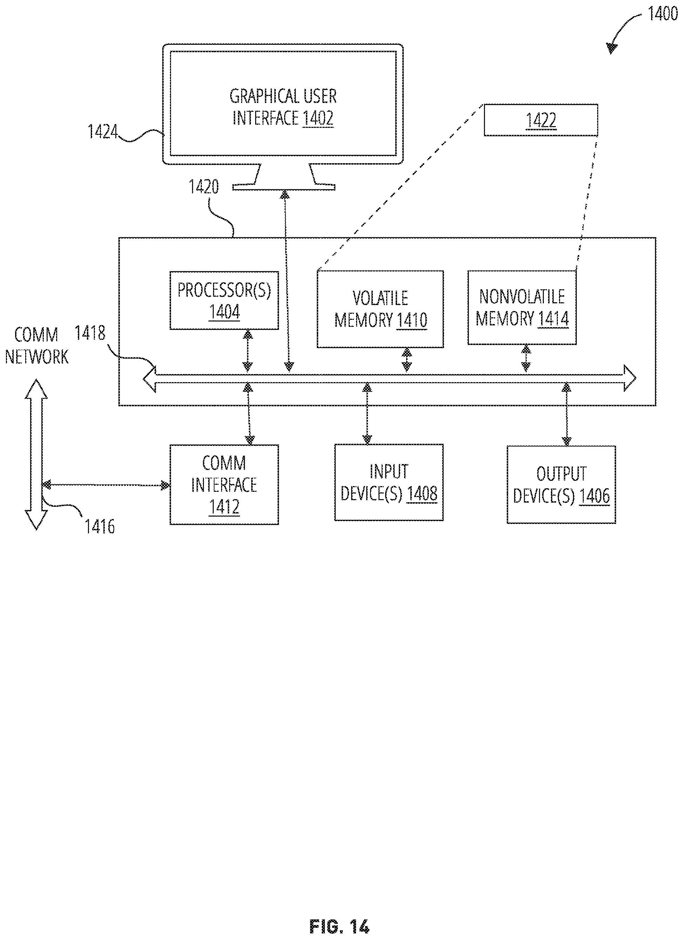

FIG. 14 is an example block diagram of a computing apparatus 1400 that may incorporate embodiments of the present invention. FIG. 14 is merely illustrative of a machine system to carry out aspects of the technical processes described herein, and does not limit the scope of the claims. One of ordinary skill in the art would recognize other variations, modifications, and alternatives. In one embodiment, the computing apparatus 1400 typically includes a graphical user interface 1402, a data processing system 1420, a communication network interface 1412, input device(s) 1408, output device(s) 1406, and the like.

As depicted in FIG. 14, the data processing system 1420 may include one or more processor(s) 1404 that communicate with a number of peripheral devices via a bus subsystem 1418. These peripheral devices may include input device(s) 1408, output device(s) 1406, communication network interface 1412, and a storage subsystem, such as memory (e.g., a volatile memory 1410 and a nonvolatile memory 1414).

The volatile memory 1410 and/or the nonvolatile memory 1414 may store computer-executable instructions and thus forming logic 1422 that when applied to and executed by the processor(s) 1404 implement embodiments of the processes disclosed herein.

The input device(s) 1408 include devices and mechanisms for inputting information to the data processing system 1420. These may include a keyboard, a keypad, a touch screen incorporated into the graphical user interface 1402, audio input devices such as voice recognition systems, microphones, and other types of input devices. In various embodiments, the input device(s) 1408 may be embodied as a computer mouse, a trackball, a track pad, a joystick, wireless remote, drawing tablet, voice command system, eye tracking system, and the like. The input device(s) 1408 typically allow a user to select objects, icons, control areas, text and the like that appear on the graphical user interface 1402 via a command such as a click of a button or the like.

The output device(s) 1406 include devices and mechanisms for outputting information from the data processing system 1420. These may include the display device 1424 configured to display/project/present the graphical user interface 1402, speakers, printers, infrared LEDs, and so on as well understood in the art.

The communication network interface 1412 provides an interface to communication networks (e.g., communication network 1416) and devices external to the data processing system 1420. The communication network interface 1412 may serve as an interface for receiving data from and transmitting data to other systems. Embodiments of the communication network interface 1412 may include an Ethernet interface, a modem (telephone, satellite, cable, ISDN), (asynchronous) digital subscriber line (DSL), FireWire, USB, a wireless communication interface such as Bluetooth or WiFi, a near field communication wireless interface, a cellular interface, and the like.

The communication network interface 1412 may be coupled to the communication network 1416 via an antenna, a cable, or the like. In some embodiments, the communication network interface 1412 may be physically integrated on a circuit board of the data processing system 1420, or in some cases may be implemented in software or firmware, such as "soft modems", or the like. The communication network 1416 can further be communicative coupled to the plate reader as described herein.

The computing apparatus 1400 may include logic that enables communications over a network using protocols such as HTTP, TCP/IP, RTP/RTSP, IPX, UDP and the like.

The volatile memory 1410 and the nonvolatile memory 1414 are examples of tangible media configured to store computer readable data and instructions to implement various embodiments of the processes described herein. Other types of tangible media include removable memory (e.g., pluggable USB memory devices, mobile device SIM cards), optical storage media such as CD-ROMS, DVDs, semiconductor memories such as flash memories, non-transitory read-only-memories (ROMS), battery-backed volatile memories, networked storage devices, and the like. The volatile memory 1410 and the nonvolatile memory 1414 may be configured to store the basic programming and data constructs that provide the functionality of the disclosed processes and other embodiments thereof that fall within the scope of the present invention.

Logic 1422 that implements embodiments of the present invention may be stored in the volatile memory 1410 and/or the nonvolatile memory 1414. Said logic 1422 may be read from the volatile memory 1410 and/or nonvolatile memory 1414 and executed by the processor(s) 1404. The volatile memory 1410 and the nonvolatile memory 1414 may also provide a repository for storing data used by the logic 1422.

The volatile memory 1410 and the nonvolatile memory 1414 may include a number of memories including a main random-access memory (RAM) for storage of instructions and data during program execution and a read only memory (ROM) in which read-only non-transitory instructions are stored. The volatile memory 1410 and the nonvolatile memory 1414 may include a file storage subsystem providing persistent (non-volatile) storage for program and data files. The volatile memory 1410 and the nonvolatile memory 1414 may include removable storage systems, such as removable flash memory.

The bus subsystem 1418 provides a mechanism for enabling the various components and subsystems of data processing system 1420 communicate with each other as intended. Although the communication network interface 1412 is depicted schematically as a single bus, some embodiments of the bus subsystem 1418 may utilize multiple distinct busses.

It will be readily apparent to one of ordinary skill in the art that the computing apparatus 1400 may be a device such as a smartphone, a desktop computer, a laptop computer, a rack-mounted computer system, a computer server, or a tablet computer device. As commonly known in the art, the computing apparatus 1400 may be implemented as a collection of multiple networked computing devices. Further, the computing apparatus 1400 will typically include operating system logic (not illustrated) the types and nature of which are well known in the art.

"Autoloader attachment" refers to an attachment for facilitating the transfer of a culture device into the plate reader so that the plate reader can capture an image of the culture device. The autoloader attachment can include one or more servos and controllers to load the culture device. In at least one embodiment, the autoloader attachment can load a sequence of culture devices sequentially without user intervention.

"Circuitry" refers to electrical circuitry having at least one discrete electrical circuit, electrical circuitry having at least one integrated circuit, electrical circuitry having at least one application specific integrated circuit, circuitry forming a general purpose computing device configured by a computer program (e.g., a general purpose computer configured by a computer program which at least partially carries out processes or devices described herein, or a microprocessor configured by a computer program which at least partially carries out processes or devices described herein), circuitry forming a memory device (e.g., forms of random access memory), or circuitry forming a communications device (e.g., a modem, communications switch, or optical-electrical equipment).

"Culture device" refers to an article adapted to house a nutrient medium that facilitates the growth of a microorganism. Optionally, the culture device may comprise a lid or cover to minimize the exposure of the nutrient medium to external contamination and/or to reduce the loss of moisture from the culture medium during incubation and/or storage. The culture device can have a growth compartment and nutrient medium contained within the culture device. A microorganic sample can be received in the growth compartment. Nonlimiting examples of culture devices include flasks, beakers, tubes, Petri dishes, multi-well plates, PETRIFILM plates, COMPACT DRY media sheets, SANITA-KUN sheets, and the like. The culture device can be agar plates or an all-in-one plating system such as 3M Petrifilm. Articles of the present invention include thin film culture devices, such as those disclosed in U.S. Pat. Nos. 4,476,226; 5,089,413, and 5,232,838; which are incorporated herein by reference in their entirety.

"Culture device identification" refers to a culture device that is defined by the worklist.

"Data field" refers to a place where you can store data. For example, a field in a data entry form or web form. The data field may contain data to be entered as well as data to be displayed.

"Data type" refers to type of data represented by a column.

"Different microorganism" refers to a second genus or species of microorganism.

"Dilution factor" refers to factor by which the stock solution is diluted. Can be represented by 1:n+1, where the second number (n+1) represents the total volume of solute + solvent.

"Dilution profile" refers to a collection of test instances for a given microorganic sample. The dilution profile can include the dilution factor for various plate types, the number of times the dilution factor is run, and the product specifications. Each profile field can correspond to a single profile test instance. The dilution profile can be associated with a plurality of profile fields and a plurality of profile test instances.

"Firmware" refers to software logic embodied as processor-executable instructions stored in read-only memories or media.

"Growth compartment" refers to the space in which the microorganisms grow. The growth compartment can include the nutrient medium, the microorganic sample, and the volume of air around them.

"Hardware" refers to logic embodied as analog or digital circuitry.

"Image" refers to an image of the culture device. Can include either the microorganic sample or can be a reference.

"Image section" refers to part of a page that displays the image and any annotations. The user can interact with the image section to create annotations or modify existing annotations.

"Logic" refers to machine memory circuits, non-transitory machine readable media, and/or circuitry which by way of its material and/or material-energy configuration comprises control and/or procedural signals, and/or settings and values (such as resistance, impedance, capacitance, inductance, current/voltage ratings, etc.), that may be applied to influence the operation of a device. Magnetic media, electronic circuits, electrical and optical memory (both volatile and nonvolatile), and firmware are examples of logic. Logic specifically excludes pure signals or software per se (however does not exclude machine memories comprising software and thereby forming configurations of matter).

"Main content area" refers to the part of the page which that is unique to that page (the part that is not shared with other pages).

"Microorganic sample" refers to a sample having microorganisms. Bacterial species of interest can be analyzed in a microorganic sample that may be derived from any source, such as a physiological fluid, e.g., blood, saliva, ocular lens fluid, synovial fluid, cerebral spinal fluid, pus, sweat, exudate, urine, mucus, mucosal tissue (e.g., buccal, gingival, nasal, ocular, tracheal, bronchial, gastrointestinal, rectal, urethral, ureteral, vaginal, cervical, and uterine mucosal membranes), lactation milk, feces or the like. Further, the sample may be derived from a body site, e.g., wound, skin, anterior nares, nasopharyngeal cavity, nasal cavities, anterior nasal vestibule, scalp, nails, outer ear, middle ear, mouth, rectum, vagina, axilla, perineum, anus, or another similar site.

Besides physiological fluids, other microorganic sample may include other liquids as well as solid(s) dissolved or suspended in a liquid medium. Samples of interest may include process streams, water, food, food ingredients, beverages, soil, plants or other vegetation, air, surfaces (e.g., walls, floors, equipment, utensils in a manufacturing plant, hospital, clinic, or home, for example), and the like. Preferably, the microorganic sample can be collected via a food matrix.

"Microorganism" refers to a microscopic organism such as a bacteria, virus, or fungus. The term microorganism can also refer to a microorganism that is targeted to be analyzed such as a microorganism bacteria that causes disease, preferably those commonly associated with food contamination, including, for example, aerobic bacteria, E. coli., coliforms, enterobacteria, yeast, mold, Staphylococcus aureus, Listeria, Campylobacter, Shigella, Salmonella, and the like.

"Navigational element" refers to a graphical user interface element to navigate between different culture devices or readings of culture devices.

"Nutrient medium" refers to a solid, liquid or semi-solid designed to support the growth of microorganisms or cells. The nutrient medium typically comprises at least one nutrient selected from the group consisting of a meat peptone, a casein peptone, a gelatin peptone, a soy peptone, a beef extract, a yeast extract, lactose, glucose, dextrose, tryptose, galactose, tryptone, a fat, a mineral, or a vitamin.

"Plate reader" refers to an instrument used to detect biological events of samples on culture device. Examples of plate readers can include thin-film plate readers such as the 3M Petrifilm Plate Reader or other agar-based plate readers.

"Plate type" refers to properties of a culture device that allows the culture device to proliferate selected microorganisms. Some plate types are non-specific to the species. Others, like the lactic acid plates, are specific to the genus but not the species.

"Plurality of profile fields" refers to a plurality of data fields in a dilution profile. Can relate to a single test instance or multiple test instances.

"Plurality of worklist fields" refers to a plurality of data fields in a worklist. Can relate to a single test instance or multiple test instances.

"Reading" refers to capturing sample data relating to the culture device. This can be performed by optical means (e.g., an image processing circuitry). The term "read" can be used to refer to the act of obtaining a reading. The reading can be performed by the plate reader at the direction of the computing apparatus. The term "reading" can be associated with the term "capturing". "Capturing" can refer to an act of the plate reader. The term "reading" can also include the analysis performed on the sample data whether the analysis is performed on the plate reader or computing apparatus.

"Ribbon tab" refers to a graphical control element in the form of a set of toolbars. Each toolbar can have multiple graphical buttons (e.g., lock button, navigational element) controlling different functionality, and data fields. Multiple ribbon tabs can be combined to form a ribbon.

"Sample data" refers to data from a reading of a culture device from a plate reader. The sample data can include images from the culture device or other data concerning the reading of the culture device.

"Sequence of culture devices" refers to a processing order of culture devices by the plate reader.

"Series of test instances" refers to a particular order of a plurality of test instances.

"Software" refers to logic implemented as processor-executable instructions in a machine memory (e.g., read/write volatile or nonvolatile memory or media).

"Test instance" refers to a single occurrence of reading a culture device.

"Worklist" refers to a list of operations to be performed by the plate reader and/or computing apparatus on one or more microorganic samples. The worklist can include a plurality of test instances.

The worklist can include a culture device identification to be read by a reader and include a plurality of fields for each culture device identification. The worklist can be associated with a plurality of dilution profiles (which can be the same or of a different type). The worklist can also be associated with a plurality of microorganic samples (which can be samples of a different type).

"Worklist field" refers to a field within the worklist.

"Worklist sequence" refers to an order to process culture devices within the worklist.

"Worklist test instance" refers to a test instance within a worklist. The worklist test instance can be associated with a plurality of worklist fields. A culture device can be prepared in accordance with a worklist test instance.

The phrases "in one embodiment", "in various embodiments", "in some embodiments", and the like are used repeatedly. Such phrases do not necessarily refer to the same embodiment. The terms "comprising", "having", and "including" are synonymous, unless the context dictates otherwise. The terms "comprises" and variations thereof do not have a limiting meaning where these terms appear in the description and claims.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As used herein, "a", "an", "the", "at least one", and "one or more" are used interchangeably. Thus, for example, a nutrient can be interpreted to mean "one or more" nutrients.

The term "and/or" means one or all of the listed elements or a combination of any two or more of the listed elements.

Also herein, the recitations of numerical ranges by endpoints include all numbers subsumed within that range (e.g., 1 to 5 includes 1, 1.5, 2, 2.75, 3, 3.80, 4, 5, etc.).

## Claims

1. A method, comprising:
receiving by a computing apparatus that is communicatively coupled to a plate reader either via a user interface on a display device of the computing apparatus or by the computing apparatus fetching from a data store via a network, a dilution profile that includes a plurality of profile fields, for a plurality of culture devices; wherein said dilution profile is data related to a series of test instances for the plurality of culture devices which are to be performed by the plate reader, wherein said each profile field of said plurality is a data field related to a property of a culture device that is to be processed by the plate reader; wherein said properties of the culture device include culture device identification or test instance identification, plate type, and dilution factor and optionally product specification;
storing the dilution profile in a memory of the computing apparatus;
receiving via the user interface a request to form a worklist for reading the plurality of culture devices in the series of test instances by the plate reader, wherein the worklist comprises a plurality of worklist fields that correspond to at least some of the plurality of profile fields in the dilution profile;
determining via the user interface, that the dilution profile applies to the worklist;
populating using the computing apparatus at least some of the plurality of worklist fields responsive to the determination that the dilution profile applies, with the plurality of profile fields for the plurality of culture devices for the series of test instances in the dilution profile from the memory; wherein said at least some of the plurality of worklist fields are data fields including device culture identification or test instance identification, plate type, dilution factor and optionally product specification for the culture devices in the series of test instances, so that the worklist comprises a series of worklist test instances and worklist fields associated with each worklist test instance and wherein worklist has at least a first test instance with a different plate type and a dilution factor from a second test instance; and
performing at least one action responsive to the populating, using the computing apparatus; wherein performing the at least one action comprises transmitting instructions to the plate reader, using the worklist, that causes image processing circuitry of said plate reader to capture an image of a culture device that is described in the worklist.

2. The method of claim 1, further comprising requesting worklist fields from the user by the computing apparatus via the user interface, relating to a culture device that is described in the worklist for any missing worklist field in the partially populated worklist.

3. The method of claim 1 or 2, wherein performing the at least one action further comprises:
receiving, by the computing apparatus, the image as sample data;
analyzing, by the computing apparatus, the sample data to determine a result using at least one of the plurality of worklist fields;
presenting the result to a user interface of a display device; and
selecting a different test instance of the worklist.

4. The method of any of claims 1 to 3, wherein performing at least one action further comprises reading, by the plate reader, the culture device associated with a worklist test instance to obtain sample data, and analyzing said sample data, with the computing apparatus or plate reader, to determine a result using the plurality of worklist fields.

5. The method of claim 4, wherein performing at least one action further comprises:
receiving by the computing apparatus from the user, via the user interface, a time condition to begin reading the culture device; and
reading the culture device at said time condition.

6. The method of claim 4, wherein performing at least one action further comprises:
determining whether a plate type of the culture device matches a plate type from the worklist test instance;
reading the culture device in response to the plate type of the culture device matching the worklist test instance.

7. The method of claim 4, wherein performing at least one action further comprises:
determining whether a worklist test instance in the worklist is completed;
responsive to said determination, receiving sample data from the plate reader for the culture device in the plurality of culture devices corresponding to the completed worklist test instance;
marking an indication in the worklist that the worklist test instance is completed in response to the reading;
analyzing the sample data using a plurality of worklist fields of the completed worklist test instance to produce a result; and
save the result into a data store.

8. The method of any of claims 1 to 7, wherein performing at least one action further comprises:
associating, via the computing apparatus, a threshold value with a worklist test instance, wherein said threshold value is a product specification for the worklist test instance;
determining, via the computing apparatus, based on sample data received from the plate reader and the plurality of worklist fields, a result;
determining via the computing apparatus, whether said result meets the threshold value; and
displaying a pass indication for the worklist test instance via the user interface based on the result meeting the threshold value.

9. The method of any of claims 1 to 8, wherein the test instances in the worklist are arranged a worklist sequence and wherein the culture devices are read in the order of said worklist sequence.

10. A system comprising:
a plate reader comprising image processing circuitry;
a computing apparatus, wherein said plate reader is communicatively coupled to said computing apparatus, said computing apparatus comprising:
a display device configured to display a user interface;
an input device communicatively coupled to said user interface to allow a user to interact with said user interface;
a processor; and
a memory storing instructions that, when executed by the processor, configure the computing apparatus to execute the method of claim 1.

11. The system of claim 10, wherein the computing apparatus is further configured to request worklist fields from the user, via the user interface, relating to the culture device that is described in the worklist for any missing worklist field from the partially populated worklist, and receive said worklist fields from the input device via the user interface.

12. The system of claim 10 or 11, wherein the instructions further configure the computing apparatus to:
receive the image as sample data;
analyze the sample data to determine a result using at least one of the plurality of worklist fields;
present the result to a user interface of the display device; and
select a different test instance of the worklist.

13. The system of claim 10 or 11, wherein the instructions further configure the computing apparatus to perform at least one action by:
determining whether a plate type of a culture device matches a plate type from a worklist test instance;
reading the culture device in response to the plate type of the culture device matching the worklist test instance.

14. The system of any of claims 10 to 13, wherein the plate reader comprises an autoloader attachment, wherein said autoloader attachment comprises a sequence of culture devices loaded therein; wherein said test instances in the worklist are arranged in a worklist sequence, wherein the worklist sequence is the same as the sequence of culture devices, wherein said autoloader attachment is configured to load the culture device to be read by image processing circuitry of the plate reader according to the worklist sequence.

15. The system of any of claims 10 to 14, further comprising a data store;
wherein performing at least one action further comprises:
determining whether the worklist test instance in the worklist is completed;
responsive to said determination, receiving sample data from the plate reader for a culture device corresponding to the completed worklist test instance;
marking an indication in the worklist that the worklist test instance is completed in response to the reading;
analyzing the sample data using the plurality of worklist fields of the completed worklist test instance to produce a result; and
save the result into the data store.

## Patentansprüche

1. Ein Verfahren, das Folgendes umfasst:
Empfangen, durch eine Computervorrichtung, die kommunikativ mit einem Plattenlesegerät gekoppelt ist, entweder über eine Benutzerschnittstelle auf einem Anzeigegerät der Computervorrichtung, oder indem die Computervorrichtung es über ein Netzwerk aus einem Datenspeicher abruft, eines Verdünnungsprofils, das eine Vielzahl von Profilfeldern umfasst, für eine Vielzahl von Kulturgeräten; wobei das genannte Verdünnungsprofil Daten sind, die in einer Beziehung mit einer Reihe von Testfällen für die Vielzahl von Kulturgeräten steht, die von dem Plattenlesegerät durchzuführen sind, wobei jedes genannte Profilfeld der genannten Vielzahl ein Datenfeld ist, das mit einer Eigenschaft eines Kulturgeräts, das von dem Plattenlesegerät verarbeitet werden soll, in Verbindung steht; wobei die genannten Eigenschaften des Kulturgeräts Kulturgerätkennung oder Testfallkennung, Plattentyp und Verdünnungsfaktor und optional Produktspezifikation einschließen;
Speichern des Verdünnungsprofils in einem Speicher der Computervorrichtung;
Empfangen, über die Benutzerschnittstelle, einer Anforderung zum Bilden einer Arbeitsliste zum Lesen der Vielzahl von Kulturgeräten in der Reihe von Testfällen durch das Plattenlesegerät, wobei die Arbeitsliste eine Vielzahl von Arbeitslistenfeldern umfasst, die mindestens einigen der Vielzahl von Profilfeldern in dem Verdünnungsprofil entsprechen;
Bestimmen, über die Benutzerschnittstelle, dass das Verdünnungsprofil auf die Arbeitsliste angewendet wird;
Befüllen, unter Verwendung der Computervorrichtung, von mindestens einigen der Vielzahl von Arbeitslistenfeldern als Reaktion auf die Bestimmung, dass das Verdünnungsprofil angewendet wird, mit der Vielzahl von Profilfeldern für die Vielzahl von Kulturgeräten für die Reihe von Testfällen in dem Verdünnungsprofil von dem Speicher; wobei die genannten mindestens einigen der Vielzahl von Arbeitslistenfeldern Datenfelder sind, die Gerätekulturkennung oder Testfallkennung, Plattentyp, Verdünnungsfaktor und optional Produktspezifikation für die Kulturgeräte in der Reihe von Testfällen einschließen, sodass die Arbeitsliste eine Reihe von Arbeitslisten-Testfällen und Arbeitslistenfeldern umfasst, die mit jedem Arbeitslisten-Testfall assoziiert sind, und wobei die Arbeitsliste mindestens einen ersten Testfall mit einem von einem zweiten Testfall verschiedenen Plattentyp und Verdünnungsfaktor aufweist; und
Durchführen mindestens einer Aktion als Reaktion auf das Befüllen unter Verwendung der Computervorrichtung; wobei das Durchführen der mindestens einen Aktion das Übermitteln von Anweisungen an das Plattenlesegerät unter Verwendung der Arbeitsliste umfasst, die bewirkt, dass die Bildverarbeitungsschaltung des genannten Plattenlesegeräts ein Bild eines in der Arbeitsliste beschriebenen Kulturgeräts aufnimmt.

2. Verfahren nach Anspruch 1, das ferner das Anfordern von Arbeitslistenfeldern von dem Benutzer durch die Computervorrichtung über die Benutzerschnittstelle bezüglich eines in der Arbeitsliste beschriebenen Kulturgeräts für ein beliebiges fehlendes Arbeitslistenfeld in der teilweise befüllten Arbeitsliste umfasst.

3. Verfahren nach Anspruch 1 oder 2, wobei das Durchführen der mindestens einen Aktion ferner Folgendes umfasst:
Empfangen, durch die Computervorrichtung, des Bilds als Probendaten;
Analysieren, durch die Computervorrichtung, der Probendaten, um unter Verwendung mindestens eines der Vielzahl von Arbeitslistenfeldern ein Ergebnis zu bestimmen;
Präsentieren des Ergebnisses auf einer Benutzerschnittstelle eines Anzeigegeräts; und
Auswählen eines anderen Testfalls der Arbeitsliste.

4. Verfahren nach einem der Ansprüche 1 bis 3, wobei das Durchführen mindestens einer Aktion ferner Folgendes umfasst: Lesen, durch das Plattenlesegerät, des Kulturgeräts, das mit einem Arbeitslisten-Testfall assoziiert ist, um Probendaten zu erhalten, und Analysieren der genannten Probendaten, mit der Computervorrichtung oder dem Plattenlesegerät, um unter Verwendung der Vielzahl von Arbeitslistenfeldern ein Ergebnis zu bestimmen.

5. Verfahren nach Anspruch 4, wobei das Durchführen mindestens einer Aktion ferner Folgendes umfasst:
Empfangen, durch die Computervorrichtung, von dem Benutzer über die Benutzerschnittstelle einer Zeitbedingung, um mit dem Lesen des Kulturgeräts zu beginnen; und
Lesen des Kulturgeräts zu der genannten Zeitbedingung.

6. Verfahren nach Anspruch 4, wobei das Durchführen mindestens einer Aktion ferner Folgendes umfasst:
Bestimmen, ob ein Plattentyp des Kulturgeräts einem Plattentyp von dem Arbeitslisten-Testfall entspricht;
Lesen des Kulturgeräts als Reaktion darauf, dass der Plattentyp des Kulturgeräts dem Arbeitslisten-Testfall entspricht.

7. Verfahren nach Anspruch 4, wobei das Durchführen mindestens einer Aktion ferner Folgendes umfasst:
Bestimmen, ob ein Arbeitslisten-Testfall in der Arbeitsliste abgeschlossen ist;
als Reaktion auf die genannte Bestimmung, Empfangen von Probendaten von dem Plattenlesegerät für das Kulturgerät in der Vielzahl von Kulturgeräten, die dem abgeschlossenen Arbeitslisten-Testfall entsprechen;
Markieren eines Hinweises in der Arbeitsliste, dass der Arbeitslisten-Testfall abgeschlossen ist, als Reaktion auf das Lesen;
Analysieren der Probendaten unter Verwendung einer Vielzahl von Arbeitslistenfeldern des abgeschlossenen Arbeitslisten-Testfalls, um ein Ergebnis zu produzieren; und
Speichern des Ergebnisses in einem Datenspeicher.

8. Verfahren nach einem der Ansprüche 1 bis 7, wobei das Durchführen mindestens einer Aktion ferner Folgendes umfasst:
Assoziieren, über die Computervorrichtung, eines Schwellenwerts mit einem Arbeitslisten-Testfall, wobei der genannte Schwellenwert eine Produktspezifikation für den Arbeitslisten-Testfall ist;
Bestimmen, über die Computervorrichtung, eines Ergebnisses auf der Grundlage der von dem Plattenlesegerät und der Vielzahl von Arbeitslistenfeldern erhaltenen Probendaten;
Bestimmen, über die Computervorrichtung, ob das genannte Ergebnis dem Schwellenwert genügt; und
Anzeigen eines Hinweises, dass der Test für den Arbeitslisten-Testfall bestanden wurde, über die Benutzerschnittstelle auf der Grundlage des Ergebnisses, das dem Schwellenwert genügt.

9. Verfahren nach einem der Ansprüche 1 bis 8, wobei die Testfälle in der Arbeitsliste in einer Arbeitslistensequenz angeordnet sind und wobei die Kulturgeräte in der Reihenfolge der genannten Arbeitslistensequenz gelesen werden.

10. Ein System, das Folgendes umfasst:
ein Plattenlesegerät, das eine Bildverarbeitungsschaltung umfasst;
eine Computervorrichtung, wobei das genannte Plattenlesegerät kommunikativ mit der genannten Computervorrichtung gekoppelt ist, wobei die genannte Computervorrichtung Folgendes umfasst:
ein Anzeigegerät, das dazu ausgelegt ist, eine Benutzerschnittstelle anzuzeigen;
ein Eingabegerät, das kommunikativ mit der genannten Benutzerschnittstelle gekoppelt ist, um die Interaktion eines Benutzers mit der genannten Benutzerschnittstelle zu ermöglichen;
einen Prozessor; und
einen Speicher, der Anweisungen speichert, die, wenn sie von dem Prozessor ausgeführt werden, die Computervorrichtung dazu auslegen, das Verfahren nach Anspruch 1 auszuführen.

11. System nach Anspruch 10, wobei die Computervorrichtung ferner dazu ausgelegt ist, Arbeitslistenfelder von dem Benutzer, über die Benutzerschnittstelle, bezüglich des in der Arbeitsliste beschriebenen Kulturgeräts für ein beliebiges fehlendes Arbeitslistenfeld von der teilweise befüllten Arbeitsliste anzufordern und die genannten Arbeitslistenfelder von dem Eingabegerät über die Benutzerschnittstelle zu empfangen.

12. System nach Anspruch 10 oder 11, wobei die Anweisungen die Computervorrichtung ferner zu Folgendem auslegen:
Empfangen des Bilds als Probendaten;
Analysieren der Probendaten, um unter Verwendung mindestens eines der Vielzahl von Arbeitslistenfeldern ein Ergebnis zu bestimmen;
Präsentieren des Ergebnisses auf einer Benutzerschnittstelle des Anzeigegeräts; und
Auswählen eines anderen Testfalls der Arbeitsliste.

13. System nach Anspruch 10 oder 11, wobei die Anweisungen die Computervorrichtung ferner dazu auslegen, mindestens eine Aktion durch Folgendes durchzuführen:
Bestimmen, ob ein Plattentyp eines Kulturgeräts einem Plattentyp von einem Arbeitslisten-Testfall entspricht;
Lesen des Kulturgeräts als Reaktion darauf, dass der Plattentyp des Kulturgeräts dem Arbeitslisten-Testfall entspricht.

14. System nach einem der Ansprüche 10 bis 13, wobei das Plattenlesegerät ein Autoloader-Zusatzgerät umfasst, wobei das genannte Autoloader-Zusatzgerät eine Sequenz von in dieses geladenen Kulturgeräten umfasst; wobei die genannten Testfälle in der Arbeitsliste in einer Arbeitslistensequenz angeordnet sind, wobei die Arbeitslistensequenz die gleiche ist wie die Sequenz von Kulturgeräten, wobei das genannte Autoloader-Zusatzgerät dazu ausgelegt ist, das Kulturgerät so zu laden, dass es von der Bildverarbeitungsschaltung des Plattenlesegeräts gemäß der Arbeitslistensequenz gelesen werden kann.

15. System nach einem der Ansprüche 10 bis 14, das ferner einen Datenspeicher umfasst;
wobei das Durchführen mindestens einer Aktion ferner Folgendes umfasst:
Bestimmen, ob der Arbeitslisten-Testfall in der Arbeitsliste abgeschlossen ist;
als Reaktion auf die genannte Bestimmung, Empfangen von Probendaten von dem Plattenlesegerät für ein Kulturgerät, das dem abgeschlossenen Arbeitslisten-Testfall entspricht;
Markieren eines Hinweises in der Arbeitsliste, dass der Arbeitslisten-Testfall abgeschlossen ist, als Reaktion auf das Lesen;
Analysieren der Probendaten unter Verwendung der Vielzahl von Arbeitslistenfeldern des abgeschlossenen Arbeitslisten-Testfalls, um ein Ergebnis zu produzieren; und
Speichern des Ergebnisses in dem Datenspeicher.

## Revendications

1. Procédé comprenant :
la réception par un appareil informatique qui est couplé en communication à un lecteur de plaques soit via une interface utilisateur sur un dispositif d'affichage de l'appareil informatique, soit par l'appareil informatique extrayant à partir d'un magasin de données via un réseau, d'un profil de dilution qui inclut une pluralité de champs de profil, pour une pluralité de dispositifs de culture ; dans lequel ledit profil de dilution est lié par des données à une série d'instances de test pour la pluralité de dispositifs de culture qui doit être effectuée par le lecteur de plaques, dans lequel ledit chaque champ de profil de ladite pluralité est un champ de données lié à une propriété d'un dispositif de culture qui doit être traitée par le lecteur de plaques ; dans lequel lesdites propriétés du dispositif de culture incluent l'identification de dispositif de culture ou l'identification d'instance de test, le type de plaque, et le facteur de dilution et facultativement la spécification de produit ;
le stockage du profil de dilution dans une mémoire de l'appareil informatique ;
la réception via l'interface utilisateur d'une demande pour former une liste de travail pour la lecture de la pluralité de dispositifs de culture dans la série d'instances de test par le lecteur de plaques, dans lequel la liste de travail comprend une pluralité de champs de liste de travail qui correspondent à au moins quelques-uns de la pluralité de champs de profil dans le profil de dilution ;
la détermination via l'interface utilisateur, que le profil de dilution s'applique à la liste de travail ;
le remplissage à l'aide de l'appareil informatique d'au moins quelques-uns de la pluralité de champs de liste de travail en réponse à la détermination que le profil de dilution s'applique, avec la pluralité de champs de profil pour la pluralité de dispositifs de culture pour la série d'instances de test dans le profil de dilution à partir de la mémoire ; dans lequel lesdits au moins quelques-uns de la pluralité de champs de liste de travail sont des champs de données incluant l'identification de culture de dispositif ou l'identification d'instance de test, le type de plaque, le facteur de dilution et facultativement la spécification de produit pour les dispositifs de culture dans la série d'instances de test, de sorte que la liste de travail comprend une série d'instances de test de liste de travail et de champs de liste de travail associés à chaque instance de test de liste de travail et dans lequel une liste de travail a au moins une première instance de test avec un type de plaque différent et un facteur de dilution à partir d'une deuxième instance de test ; et
l'exécution d'au moins une action en réponse au remplissage, à l'aide de l'appareil informatique ; dans lequel l'exécution de l'au moins une action comprend la transmission d'instructions au lecteur de plaques, à l'aide de la liste de travail, qui amène des circuits de traitement d'image dudit lecteur de plaques à capturer une image d'un dispositif de culture qui est décrit dans la liste de travail.

2. Procédé selon la revendication 1, comprenant en outre une demande de champs de liste de travail à l'utilisateur par l'appareil informatique via l'interface utilisateur, concernant un dispositif de culture qui est décrit dans la liste de travail pour tout champ de liste de travail manquant dans la liste de travail partiellement remplie.

3. Procédé selon la revendication 1 ou 2, dans lequel l'exécution de l'au moins une action comprend en outre :
la réception, par l'appareil informatique, de l'image sous forme de données d'échantillon ;
l'analyse, par l'appareil informatique, des données d'échantillon pour déterminer un résultat à l'aide d'au moins l'un de la pluralité de champs de liste de travail ;
la présentation du résultat à une interface utilisateur d'un dispositif d'affichage ; et
la sélection d'une instance de test différente de la liste de travail.

4. Procédé selon l'une quelconque des revendications 1 à 3, dans lequel l'exécution d'au moins une action comprend en outre la lecture, par le lecteur de plaques, du dispositif de culture associé à une instance de test de liste de travail pour obtenir des données d'échantillon, et l'analyse desdites données d'échantillon, avec l'appareil informatique ou le lecteur de plaques, pour déterminer un résultat à l'aide de la pluralité de champs de liste de travail.

5. Procédé selon la revendication 4, dans lequel l'exécution d'au moins une action comprend en outre :
la réception par l'appareil informatique de l'utilisateur, via l'interface utilisateur, d'une condition de temps pour commencer la lecture du dispositif de culture ; et
la lecture du dispositif de culture à ladite condition de temps.

6. Procédé selon la revendication 4, dans lequel l'exécution d'au moins une action comprend en outre :
le fait de déterminer si un type de plaque du dispositif de culture correspond à un type de plaque de l'instance de test de liste de travail ;
la lecture du dispositif de culture en réponse au type de plaque du dispositif de culture correspondant à l'instance de test de liste de travail.

7. Procédé selon la revendication 4, dans lequel l'exécution d'au moins une action comprend en outre :
le fait de déterminer si une instance de test de liste de travail dans la liste de travail est terminée ;
en réponse à ladite détermination, la réception de données d'échantillon du lecteur de plaques pour le dispositif de culture dans la pluralité de dispositifs de culture correspondant à l'instance de test de liste de travail terminée ;
le marquage d'une indication dans la liste de travail que l'instance de test de liste de travail est terminée en réponse à la lecture ;
l'analyse des données d'échantillon à l'aide d'une pluralité de champs de liste de travail de l'instance de test de liste de travail terminée pour produire un résultat ; et
la sauvegarde du résultat dans un magasin de données.

8. Procédé selon l'une quelconque des revendications 1 à 7, dans lequel l'exécution d'au moins une action comprend en outre :
l'association, via l'appareil informatique, d'une valeur de seuil avec une instance de test de liste de travail, dans lequel ladite valeur de seuil est une spécification de produit pour l'instance de test de liste de travail ;
la détermination, via l'appareil informatique, sur la base de données d'échantillon reçues du lecteur de plaques et de la pluralité de champs de liste de travail, d'un résultat ;
le fait de déterminer via l'appareil informatique si ledit résultat atteint la valeur de seuil ; et
l'affichage d'une indication de réussite pour l'instance de test de liste de travail via l'interface utilisateur sur la base que le résultat atteint la valeur de seuil.

9. Procédé selon l'une quelconque des revendications 1 à 8, dans lequel les instances de test dans la liste de travail sont agencées dans une séquence de liste de travail et dans lequel les dispositifs de culture sont lus dans l'ordre de ladite séquence de liste de travail.

10. Système comprenant :
un lecteur de plaques comprenant des circuits de traitement d'image ;
un appareil informatique, dans lequel ledit lecteur de plaques est couplé en communication audit appareil informatique, ledit appareil informatique comprenant :
un dispositif d'affichage configuré pour afficher une interface utilisateur ;
un dispositif d'entrée couplé en communication à ladite interface utilisateur pour permettre à un utilisateur d'interagir avec ladite interface utilisateur ;
un processeur ; et
une mémoire stockant des instructions qui, lorsqu'elles sont exécutées par le processeur, configurent l'appareil informatique pour exécuter le procédé selon la revendication 1 .

11. Système selon la revendication 10, dans lequel l'appareil informatique est configuré en outre pour demander des champs de liste de travail à l'utilisateur, via l'interface utilisateur, concernant le dispositif de culture qui est décrit dans la liste de travail pour tout champ de liste de travail manquant de la liste de travail partiellement remplie, et recevoir lesdits champs de liste de travail du dispositif d'entrée via l'interface utilisateur.

12. Système selon la revendication 10 ou 11, dans lequel les instructions configurent en outre l'appareil informatique pour :
recevoir l'image sous forme de données d'échantillon ;
analyser les données d'échantillon pour déterminer un résultat à l'aide d'au moins l'un de la pluralité de champs de liste de travail ;
présenter le résultat à une interface utilisateur du dispositif d'affichage ; et
sélectionner une instance de test différente de la liste de travail.

13. Système selon la revendication 10 ou 11, dans lequel les instructions configurent en outre l'appareil informatique pour exécuter au moins une action en :
déterminant si un type de plaque d'un dispositif de culture correspond à un type de plaque d'une instance de test de liste de travail ;
lisant le dispositif de culture en réponse au fait que le type de plaque du dispositif de culture correspond à l'instance de test de liste de travail.

14. Système selon l'une quelconque des revendications 10 à 13, dans lequel le lecteur de plaques comprend un accessoire de chargeur automatique, dans lequel ledit accessoire de chargeur automatique comprend une séquence de dispositifs de culture chargés dans celui-ci ; dans lequel lesdites instances de test dans la liste de travail sont agencées dans une séquence de liste de travail, dans lequel la séquence de liste de travail est la même que la séquence de dispositifs de culture, dans lequel ledit accessoire de chargeur automatique est configuré pour charger le dispositif de culture devant être lu par des circuits de traitement d'image du lecteur de plaques selon la séquence de liste de travail.

15. Système selon l'une quelconque des revendications 10 à 14, comprenant en outre un magasin de données :
dans lequel l'exécution d'au moins une action comprend en outre :
le fait de déterminer si l'instance de test de liste de travail dans la liste de travail est terminée ;
en réponse à ladite détermination, la réception de données d'échantillon du lecteur de plaques pour un dispositif de culture correspondant à l'instance de test de liste de travail terminée ;
le marquage d'une indication dans la liste de travail que l'instance de test de liste de travail est terminée en réponse à la lecture ;
l'analyse des données d'échantillon à l'aide de la pluralité de champs de liste de travail de l'instance de test de liste de travail terminée pour produire un résultat ; et
la sauvegarde du résultat dans le magasin de données.
